# EUROPÄISCHE PATENTSCHRIFT

(11) **EP 0 966 985 B1**
(45) Veröffentlichungstag und Bekanntmachung des Hinweises auf die Patenterteilung: **31.03.2004**
(21) Anmeldenummer: 99111746.6
(22) Anmeldetag: 17.06.1999
(51) Int. Cl.: A61M 39/14

(54) **Konnektorelement mit Verschlussteil insbesondere zum Verbinden von medizinischen Schläuchen, Kanülen und Kathetern**
Connecting element with sealing stopper in particular for connecting medical tubes, cannulae and catheters
Elément de connection avec obturateur scellable en particulier pour la connection de tubes médicaux, canules et cathéters

(30) Priorität: 26.06.1998 DE 19828651
(43) Veröffentlichungstag der Anmeldung: 29.12.1999
(73) Patentinhaber: Fresenius Medical Care Deutschland GmbH, 61352 Bad Homburg v.d.H. (DE)
(72) Erfinder: Lauer, Martin, 66606 St. Wendel (DE)
(74) Vertreter: Gossel, Hans K., Dipl.-Ing.

(56) Entgegenhaltungen:
- DE-A- 2 830 800
- DE-A- 2 846 677
- US-A- 4 019 512
- US-A- 4 030 494
- US-A- 4 195 632
- US-A- 5 492 147

## Beschreibung

Die vorliegende Erfindung betrifft ein Konnektorelement insbesondere zum Verbinden von Schläuchen, Kanülen und Kathetern mit einem Bereich zur Führung eines durchströmendes Mediums, mit einem relativ zu diesem Bereich bewegbaren Verschlussteil, durch das der Bereich gegenüber der das Konnektorelement umgebenden Atmosphäre abdichtbar ist, und das von diesem Bereich aufgrund eines zwischen dem Bereich und dem Verschlussteil angeordneten Öffnungselementes beabstandet ist.

Ein wichtiges Verwendungsgebiet derartiger Konnektoren ist die Verbindung von Schläuchen, Kanülen und Kathetern sowie von Vorratsbehältern mit medizinischen Arbeitsmitteln, beispielsweise mit entsprechenden Anschlußelementen eines Dialysegerätes. Um eine Gefährdung von Patienten sicher auszuschließen, werden hohe Anforderungen an die Ausführung und Qualität der Konnektoren sowie an die Sterilität der herzustellenden Verbindung gestellt. Dabei muß sichergestellt werden, daß vor, während und nach dem Konnektionsvorgang eine Kontamination medienberührter Teile ausgeschlossen wird.

Aus der Patentschrift U.S. 4,030,494 ist ein gattungsgemäßes Konnektorelement bekannt. Das in dieser Druckschrift offenbarte Verschlussteil des Konnektorelementes weist eine Membran auf, die in dem zu der Konnektionsöffnung liegenden Endbereich des Verschlussteils angeordnet ist. Eine derartige Anordnung hat zum einen den Nachteil, dass ein wirksamer Berührschutz nicht gegeben ist. Darüber hinaus besteht ein weiterer Nachteil darin, dass während des Konnektionsvorgangs die Membran selbst berührt wird. Aufgrund der unmittelbaren Kontaktierung der Membran zum Zwecke des Konnektionsvorganges kann eine Kontamination der Membranaußenseite nicht sicher ausgeschlossen werden. Ist eine der Membranen vor der Konnektion kontaminiert, wird diese Verunreinigung auf die andere Membran übertragen und gelangt unter Umständen im geöffneten Zustand der Membran auf die Membraninnenseite und somit in Bereiche, die steril zu halten sind.

Die Patentschrift 5,492,147 offenbart ein Konnektorelement mit einem Verschlussteil, dessen Membran sich an dem zur Konnektionsöffnung des Konnektorelementes gerichteten Endbereich des Grundkörpers befindet. Auch bei dieser Ausführungsform findet ein unmittelbarer Membrankontakt während des Konnektionsvorgangs statt.

Aus der Druckschrift DE-OS 28 46 677 geht ein Konnektorelement hervor, dass in seinem Endbereich von einer zu durchstoßenden Membran begrenzt ist. Das Durchstoßen der Membran erfolgt durch einen Aufstoßdom des jeweils anderen Konnektorelementes. Ein relativ zu dem Bereich zur Führung eines durchströmenden Mediums bewegbares Verschlussteil ist nicht vorgesehen.

Aus der Patentschrift U.S. 3,986,508 sind Konnektorelemente bekannt, die vor der Benutzung mit einem Verschlußteil versehen werden, um auf diese Weise zu erreichen, daß die mediendurchströmten Bereiche der Konnektoren gegenüber der Atmosphäre abgedichtet und somit vor einer entsprechenden Kontamination geschützt sind. Die Verschlußteile werden vor Benutzung in die Konnektorelemente abdichtend eingesetzt. Anschließend werden die Konnektorelemente in einem Wärmesterilisator in einem teilweise zusammengefügten Zustand sterilisiert. Im Anschluß an diesen Vorgang werden die Konnektorelemente derart zusammengeführt, daß die Verschlußteile durch einen in einem der Konnektorelemente vorgesehenen Dom durchstoßen werden und somit eine Durchführung für das zu fördernde Medium geschaffen wird. Dabei durchstößt die Spitze des Doms zunächst die Innenseite des Verschlußteils eines Konnektorelementes und anschließend das benachbart angeordnete Verschlußteil des anderen Konnektorelementes. Einer derartigen Anordnung ist es nachteilig, daß eine Kontamination, die sich auf der Seite des dem Dorn zugewandten Verschlußteils befindet, beim Durchstoßen der Verschlußteile in einen medienbeaufschlagten Bereich und somit möglicherweise in das durch den Konnektor geführte Medium gelangt.

Es ist die Aufgabe der vorliegenden Erfindung einen gattungsgemäßen Konnektor dahingehend weiterzubilden, daß eine sterile Verbindungsbildung gewährleistet wird.

Diese Aufgabe wird dadurch gelöst, dass das Verschlussteil einen Grundkörper umfasst, über dessen Querschnitt sich die Membran erstreckt und der einen zur Konnektionsöffnung des Konnektorelementes gerichteten Endbereich aufweist, wobei die Membran von diesem Endbereich des Grundkörpers entfernt angeordnet ist.

Das Verschlussteil ist als relativ zu den Bereichen zur Führung des Mediums bewegbare Kappe ausgeführt, deren Membran zum Zwecke einer Durchführung der Flüssigkeit während der Konnektion geöffnet werden muss. Erfindungsgemäß erfolgt das Öffnen der Membran durch ein Öffnungselement des Konnektorelementes.

Das Konnektorelement weist ein Öffnungselement auf, das derart ausgeführt ist, daß das Verschlußteil weder in der geschlossenen noch in einer geöffneten Position mit dem Bereich zur Führung des Mediums in Berührung kommt. Dadurch wird erreicht, daß die Öffnung des Verschlußteils während der Konnektion nicht durch die Bereiche erfolgt, durch die das Medium geführt wird, sondern durch Öffnungselemente, die derart ausgeführt bzw. angeordnet sind, daß das Verschlußteil mit den Bereichen zur Führung des Mediums weder im geschlossenen noch in einer beliebigen geöffneten Position in Berührung kommt. Dadurch wird stets eine sterile Verbindung zwischen beiden Konnektorelementen erreicht. Dies gilt auch für den Fall, daß Kontaminate möglicherweise bis auf die Innenseite des Verschlußteils gelangt sind. Auch in diesem Fall erfolgt eine sterile Verbindungsbildung im medienberührten Bereich. Wird beispielsweise ein Verschlußteil während der Konnektion durch eines der Konnektorelemente in Richtung eines medienberührten Bereiches bewegt, erfolgt die für die Konnektion erforderliche Öffnung des Verschlußteils derart, daß mittels des Öffnungselementes eine Berührung mit den medienberührten Teilen des Konnektors ausgeschlossen wird.

In weiterer Ausgestaltung der vorliegenden Erfindung ist vorgesehen, daß der Grundkörper zylindrisch ausgeführt ist und die Membran in einem der Endbereiche des Grundkörpers angeordnet ist. Ein derartiges Verschlußteil wird vorteilhaft derart eingesetzt, daß der Endbereich des Grundkörpers, der die Membran aufweist, in Richtung auf das Öffnungselement des Konnektorelementes ausgerichtet ist. Daraus ergibt sich der Vorteil, daß ein wesentlicher Teil des Grundkörpers einen Berührschutz darstellt, durch den die Membran von den Endbereichen der Konnektorelemente in etwa um die Länge des Grundkörpers zurückversetzt ist, was dazu führt, daß eine versehentliche Berührung der Membran bei entsprechender Ausführung des Durchmessers des Grundkörpers ausgeschlossen werden kann.

In weiterer Ausgestaltung der vorliegenden Erfindung ist vorgesehen, daß die Membran zum Zwecke der Öffnung des Konnektors einen geradlinigen oder kreuzförmigen Schlitz aufweist. Dieser Schlitz ist derart ausgeführt, daß im diskonnektierten Zustand der Konnektorelemente eine Abdichtung der Bereiche zur Führung des Mediums erfolgt und erst bei der Betätigung der Konnektorelemente bzw. bei deren Zusammenführen eine Öffnung des Schlitzes erfolgt, die schließlich die Durchströmung des Konnektors ermöglicht.

Gemäß einer bevorzugten Ausgestaltung der vorliegenden Erfindung ist die Membran als Silikonmembran ausgeführt. Eine derartige Membran schließt sich nach dem Schlitzen mit einer ausreichenden Dichtigkeit und läßt sich andererseits leicht aufklappen und durchdringen.

Gemäß einer bevorzugten Ausgestaltung der vorliegenden Erfindung umfaßt der Bereich zur Führung eines durchströmenden Mediums einen Rohrstutzen. Der Rohrstutzen begrenzt die medienberührten Teile des Konnektorelementes und kann bei der Konnektion fluiddicht in den Rohrstutzen eines zu verbindenden Konnektorelementes eingeführt werden.

Besonders vorteilhaft ist es, wenn das Öffnungselement einen Rohrstutzen darstellt, der den Bereich zur Führung des Mediums umgibt.

Der Bereich zur Führung des Mediums sowie das Öffnungselement können durch konzentrisch angeordnete Rohrstutzen gebildet werden, wobei der das Öffnungselement bildende Rohrstutzen mit dem den Bereich zur Führung des Mediums begrenzenden Rohrstutzen abschließt oder geringfügig über diesen übersteht. Dadurch wird eine verhältnismäßig einfache Anordnung erreicht, wobei das äußere der konzentrischen Rohre das Öffnungselement bildet und das innere konzentrische Rohr den Bereich zur Führung des durchströmenden Mediums begrenzt. Erfindungsgemäß wird die notwendige Öffnung des Verschlußteils durch die Öffnungselemente dadurch erreicht, daß diese außen liegend angeordnet sind und dadurch eine Öffnung des bewegten Verschlußteils bewirken, bevor es zu einer Berührung der medienbeaufschlagten Bereiche kommt. Dabei ist es vorteilhaft, wenn der Rohrstutzen des Öffnungselementes geringfügig über den Rohrstutzen des Bereiches zur Führung des Mediums übersteht oder mit diesem abschließt.

Wesentlich ist in beiden Fällen, daß das Verschlußteil derart geführt bzw. geöffnet wird, daß in allen Stadien der Bewegung des Verschlußteils eine Berührung mit den Bereichen zur Führung des Mediums, insbesondere mit den Endbereichen der Rohrstutzen, die die Bereiche zur Führung des Mediums begrenzen, vermieden wird. Bei einer gekrümmten Ausführung der Membran ist es ebenso möglich, daß der das Öffnungselement bildende Rohrstutzen gegenüber dem Rohrstutzen des Bereiches zur Aufnahme des Mediums zurückversetzt ist.

In weiterer Ausgestaltung der vorliegenden Erfindung ist vorgesehen, daß das Konnektorelement ein Gehäuse aufweist und der Bereich zur Führung des Mediums sowie das Öffnungselement in dem Gehäuse aufgenommen sind.

Besonders vorteilhaft ist es, wenn sich zwischen Gehäuse und Öffnungselement ein Ringspalt erstreckt, in dem das Verschlußteil bewegbar aufgenommen ist. Entsprechend wird das Verschlußteil während der Konnektion zwischen dem Gehäuse und der Wandung des Öffnungselementes geführt und durch das Öffnungselement in eine Position bewegt, durch das das Verschlußteil geöffnet wird. Im Anschluß daran oder auch gleichzeitig kann das Verschlußteil des anderen Konnektorelementes ebenfalls in einem Ringspalt in äquivalenter Weise zurückbewegt werden, so daß letztlich die Bereiche zur Führung des Mediums miteinander in Kontakt treten können, ohne daß die Verschlußteile auf ihrer Außen- oder Innenseite berührt werden.

Gemäß einer bevorzugten Ausgestaltung der vorliegenden Erfindung ist vorgesehen, daß das Verschlußteil in dem Gehäuse mittels einer Arretierung fixierbar ist. Dadurch wird es möglich, die Konnektoren sowie die Verschlußteile in zwei getrennten Arbeitsgängen herzustellen und nach der Herstellung die Verschlußteile in die Gehäuse einzusetzen und an der gewünschten Stelle mittels der Arretierung zu fixieren. Die Arretierung kann in einem sich vom Gehäuse erstreckenden Vorsprung und in einer entsprechenden Aussparung im Verschlußteil bestehen.

In weiterer Ausgestaltung der vorliegenden Erfindung ist vorgesehen, daß der Bereich zur Führung des Mediums durch ein Absperrelement verschlossen ist. Das Absperrelement bewirkt den fluiddichten Abschluß der Bereiche zur Führung des Mediums und kann bei Bedarf, d.h. bei der Konnektion, durch entsprechende Durchstoßelemente geöffnet werden. Dabei ist es möglich, daß der Bereich beispielsweise von einem Vorratsbehälter bis zu dem Absperrelement flüssigkeitsgefüllt ist.

Besonders vorteilhaft ist es, wenn ein relativ zu dem Absperrelement bewegbarer Durchstoßkörper vorgesehen ist, der derart angeordnet ist, daß das Absperrelement durch den Durchstoßkörper geöffnet werden kann. Der Durchstoßkörper kann dabei während der Konnektion durch eines der Konnektorelemente derart bewegt werden, daß zunächst eines der Absperrelemente zweier zu verbindender Konnektorelemente und im Anschluß daran das Absperrelement des anderen Konnektorelementes durchstoßen wird und schließlich die Durchströmung des Konnektors ermöglicht wird.

In weiterer Ausgestaltung der Erfindung ist vorgesehen, daß der Bereich zur Führung des Mediums durch einen Rohrstutzen gebildet wird und der Durchstoßkörper in dem Rohrstutzen angeordnet ist. Dabei erfolgt die Montage eines derartigen Konnektorelementes dergestalt, daß zunächst der Durchstoßkörper an einer geeigneten Position in den Rohrstutzen eingeführt wird und anschließend die Verschlußteile eingesetzt werden.

Der Durchstoßkörper kann einen Vorsprung aufweisen, der mit einem Rohrstutzen verbindbar ist. Dabei wird der Vorsprung des Durchstoßkörpers während der Konnektion von einem der Konnektorelemente berührt, wodurch der Durchstoßkörper während der weiteren Konnektion bewegt wird und dadurch ein Absperrelement geöffnet wird.

Gemäß einer bevorzugten Ausgestaltung der vorliegenden Erfindung ist vorgesehen, daß das Absperrelement als Spritzgußmembran ausgeführt ist. Dabei findet vorzugsweise der Werkstoff Polypropylen Anwendung. Durch die Verwendung von Spritzgußmembranen werden die entsprechenden Bereiche der Konnektorelemente hermetisch dicht, totraumfrei und monomaterial verschlossen. Ein besonderer Vorteil der Verwendung von Spritzgußmembranen ist die Tatsache, daß diese in einem Arbeitsgang gemeinsam mit den übrigen Bestandteilen des Konnektorelementes spritzgegossen werden können, wodurch eine nachträgliche Einschweißung von Folienmembranen oder nachträgliche Anschweißung oder Verschnappung von membranbestückten Einzelbauteilen entfallen kann.

Weitere Einzelheiten und Vorteile werden anhand eines in der Zeichnung dargestellten Ausführungsbeispiels näher erläutert. Es zeigen:
- Fig. 1:: Eine Schnittdarstellung durch die erfindungsgemäßen Konnektorelemente in einer Grundstellung vor der Konnektion,
- Fig. 2:: die Konnektorelemente gemäß Fig. 1 nach dem Einführen des rechts dargestellten Konnektorelementes bis zum Berühren des Verschlußteils des linken Konnektorelementes,
- Fig. 3:: die Konnektorelemente gemäß Fig. 1 nach dem Öffnen des Verschlußteils des links dargestellten Konnektorelementes,
- Fig. 4:: die Konnektorelemente gemäß Fig. 1 nach dem Öffnen beider Verschlußteile,
- Fig. 5:: die Konnektorelemente gemäß Fig. 1 nach dem weiteren Einführen der Konnektorelemente bis zum Anliegen der Membran des rechten Konnektorelementes an dem Durchstoßkörper,
- Fig. 6:: die Konnektorelemente gemäß Fig. 1 nach dem Öffnen des Absperrelementes des rechts dargestellten Konnektorelementes,
- Fig. 7:: die Konnektorelemente gemäß Fig. 1 nach dem Öffnen beider Absperrelemente und
- Fig. 8:: eine Schnittdarstellung durch ein erfindungsgemäßes Konnektorelement mit als Dorn ausgeführtem Durchstoßkörper.

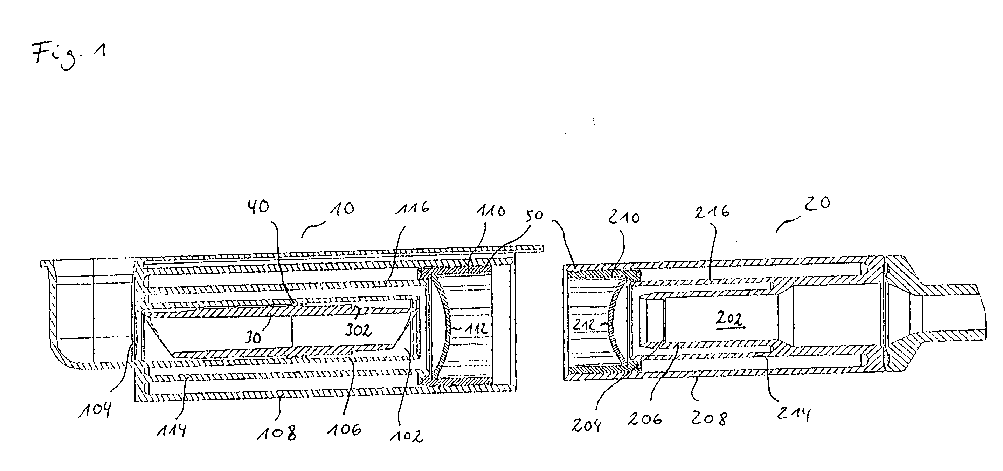

Fig. 1 zeigt in einer Schnittdarstellung die Konnektorelemente 10, 20. Beide Konnektorelemente 10, 20 weisen ein Gehäuse 108, 208 auf, das jeweils auf der einen Seite einen Anschluß für einen Schlauch oder sonstigen Arbeitsmittel und auf der anderen Seite eine Konnektionsöffnung aufweist. In dem zu der Konnektionsöffnung gerichteten Seite der Konnektorelemente 10, 20 befindet sich jeweils ein Verschlußteil 110, 210. Die Verschlußteile 110, 210 bestehen aus einem im wesentlichen zylindrischen Grundkörper, in dessen einen Endbereich sich die Membran 112, 212 erstreckt. Der andere Endbereich ist vorteilhaft von der Membran derart entfernt angeordnet, daß ein wirksamer Berührschutz der Membran gewährleistet wird. Dies wird dadurch erreicht, daß die Membranen 112, 212 von den zur Konnektionsöffnung gerichteten Endbereichen des zylindrischen Grundkörpers entfernt angeordnet sind.

Die Grundkörper 110, 210 werden mittels der Arretierung 50 an einer gewünschten Position des Gehäuses 108, 208 fixiert. Daraus ergibt sich der Vorteil, daß die Anordnung der Verschlußteile 110, 210 exakt an der gewünschten Position erfolgt. Die Arretierung 50 besteht aus einem sich auf der Innenseite des Gehäuses 108, 208 erstreckenden Vorsprung sowie aus einem auf der Außenseite des zylindrischen Grundkörpers der Verschlußteile 110, 210 befindlichen Nut.

In dem Gehäuse 108, 208 ist jeweils ein Öffnungselement 114, 214 angeordnet, das gemäß dem vorliegenden Ausführungsbeispiel aus Rohrstutzen 116, 216 besteht. Diese Rohrstutzen 116, 216 begrenzen zwischen ihrer Außenfläche und der Innenfläche des Gehäuses 108, 208 einen Ringspalt, in den das bewegbare Verschlußteil 110, 210 verschieblich aufgenommen ist.

Die Bereiche 102, 202 zur Aufnahme eines durchströmenden Mediums werden durch die Rohrstutzen 106, 206 begrenzt, die sich konzentrisch und innenliegend zu den Rohrstutzen 116, 216 der Öffnungselemente 114, 214 erstrecken. Der Bereich 102 des links dargestellten Konnektorelementes 10 ist durch eine als Absperrelement 104 dienende Membran verschlossen. Ein entsprechendes Absperrelement 204 findet sich ebenfalls im Rohrstutzen 206 des rechts dargestellten Konnektorelementes 20.

In der in Fig. 1 dargestellten Grundposition sind die Membranen 112, 212 der Verschlußteile 110, 210 geschlossen und ermöglichen einen dichten Abschluß insbesondere der Bereiche 102, 202 zur Führung des durchströmenden Mediums. Somit können die entsprechenden Bereiche 102, 202 beispielsweise vor einem Anhusten bzw. vor der Beaufschlagung mit flüssigen oder festen Partikeln wirksam geschützt werden.

Wie aus Fig. 1 ersichtlich, stehen die Enden der Rohrstutzen 116, 216 geringfügig über die Rohrstutzen 106, 206 über. Dadurch wird es möglich, beim Bewegen der Verschlußteile 110, 210 eine Öffnung der Membranen 112, 212 zu erreichen, bevor diese mit den Bereichen 102, 202 bzw. mit den Rohrstutzen 106, 206 in Berührung kommen. Dadurch wird gewährleistet, daß selbst für den Fall, daß sich auf der jeweiligen Innenseite der Membranen 112, 212 Erreger oder sonstige Partikel befinden, eine sterile Konnektion ermöglicht wird, da erfindungsgemäß eine Berührung der Membranen 112, 212 mit den Bereichen 102, 202 bzw. insbesondere mit den Endbereichen der Rohrstutzen 106, 206 wirksam verhindert wird.

In dem Rohrstutzen 106 des Konnektorelementes 10 ist ein Durchstoßkörper 30 angeordnet, der in seinem mittleren Bereich mittels einer Arretierung 40 an einer geeigneten Position in dem Rohrstutzen 106 gehalten wird. Der Durchstoßkörper 30 weist einen sich am Außenumfang erstreckenden Vorsprung 302 auf.

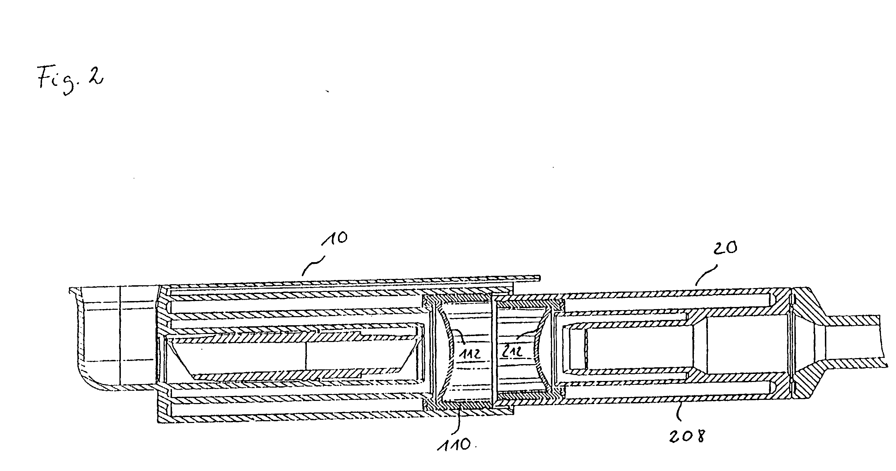

Fig. 2 zeigt die erfindungsgemäßen Konnektorelemente 10, 20 in einem Zustand, in dem sich das Gehäuse 208 des Konnektorelementes 20 sowie das Verschlußteil 110 jeweils in ihren Stirnflächen berühren. Eine Bewegung der Verschlußteile 110, 210 hat noch nicht stattgefunden und die Membranen 112, 212 sind noch geschlossen.

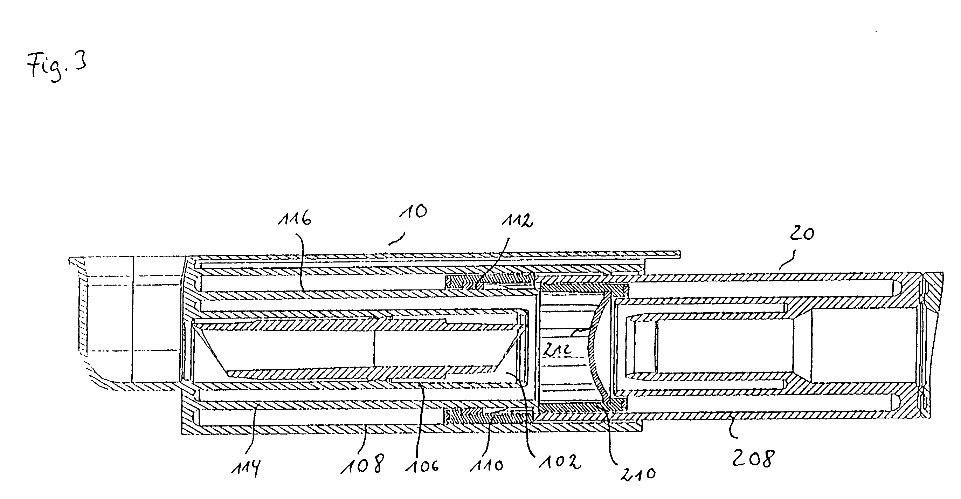

Ausgehend von dieser in Fig. 2 dargestellten Position zeigt Fig. 3 einen Zustand, bei dem das Konnektorelement 20 weiter in das Konnektorelement 10 eingeführt wurde. Dabei nimmt das Gehäuse 108 des Konnektorelementes 10 das Gehäuse 208 des Konnektorelementes 20 auf. Die stirnseitige Berührung des Gehäuses 208 mit dem Verschlußteil 110 bzw. die Bewegung des Konnektorelementes 20 bewirkt, daß das Verschlußteil 110 in den Ringspalt geschoben wird, der sich zwischen Gehäuse 108 und dem das Öffnungselement 114 bildenden Rohrstutzen 116 erstreckt. Dabei wird die Membran 112 von dem Endbereich des Rohrstutzens 116 entfernt gehalten, so daß zu keinem Konnektionsstadium eine Berührung erfolgen kann. Die geöffneten Membranteile werden ebenfalls in dem Ringspalt aufgenommen, ohne daß vor oder während der Öffnung deren Kontakt mit dem Bereich 102 bzw. dem Rohrstutzen 106 und insbesondere mit dem Endbereich des Rohrstutzens 106 erfolgt. Dadurch wird erreicht, daß unabhängig davon, ob auf der Innenoder Außenseite der Membran 112 eine Kontamination vorliegt, eine Berührung der Membran 112 und damit die Kontamination eines mediengeführten Bereiches sicher vermieden wird.

In der in Fig. 3 dargestellten Position ist die Membran 112 des Öffnungselementes 110 des Konnektorelementes 10 bereits geöffnet, während die Membran 212 des Verschlußteils 210 des Konnektorelementes 20 noch verschlossen ist. Die Endbereiche des Rohrstutzens 116 und des Verschlußteils 210 liegen in dieser Position stirnseitig aneinander.

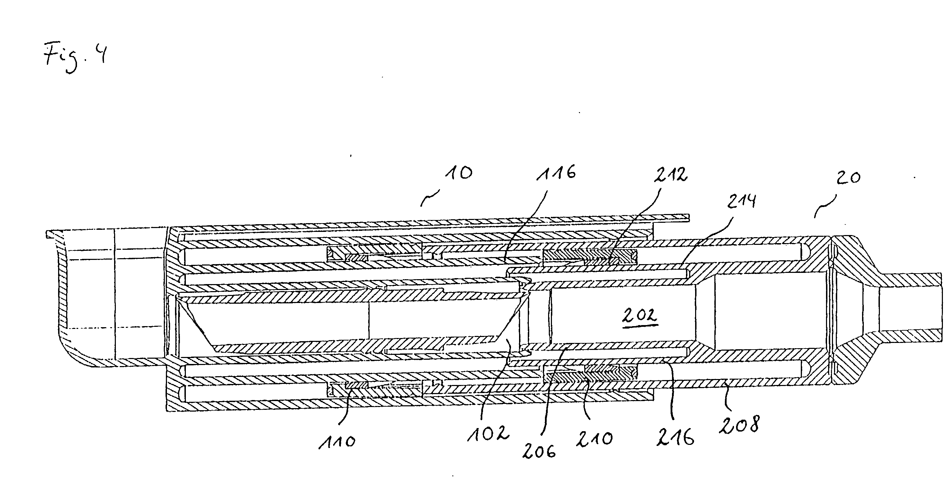

Fig. 4 zeigt die Position der Konnektorelemente 10,20, bei der das Konnektorelement 20 ausgehend von der in Fig. 3 dargestellten Position weiter in das Konnektorelement 10 eingeführt wurde. Diese Einführbewegung hat zur Folge, daß einerseits das geöffnete Verschlußteil 110 weiter in den Ringspalt eingeschoben wird. Andererseits führt die Bewegung dazu, daß durch den Kontakt des Rohrstutzens 116 mit dem Verschlußteil 210 ein Verschieben dieses Verschlußteils 210 in den Ringspalt zwischen Rohrstutzen 216 und Gehäuse 208 des Konnektorelementes 20 bewirkt wird. Insbesondere führt die Bewegung dazu, daß die Membran 212 des Verschlußteils 210 durch den Endbereich des als Öffnungselement 214 dienenden Rohrstutzens 216 derart geöffnet wird, daß auch diese Membran 212 nicht mit den medienberührten Teilen insbesondere nicht mit dem Endbereich des Rohrstutzens 206 des Konnektorelementes 20 in Kontakt kommt.

Insgesamt ergibt sich damit die Möglichkeit, daß die Rohrstutzen 106, 206 miteinander in Kontakt treten, ohne daß auch nur ein Teil der Rohrstutzen 106,206 oder ein anderer medienbeaufschlagter Bereich 102, 202 mit den Verschlußteilen 110, 210 bzw. den Membranen 112, 212 in Berührung getreten ist.

Aus Fig. 4 wird weiter ersichtlich, daß das Öffnungselement 214 sowie der Rohrstutzen 206 des Konnektorelementes 20 in die entsprechenden Teile des Konnektorelementes 10 eingeführt werden. Ebenso ist es jedoch möglich, daß die Rohrstutzen des Konnektorelementes 10 in die entsprechenden Rohrstutzen des Konnektorelementes 20 aufgenommen werden.

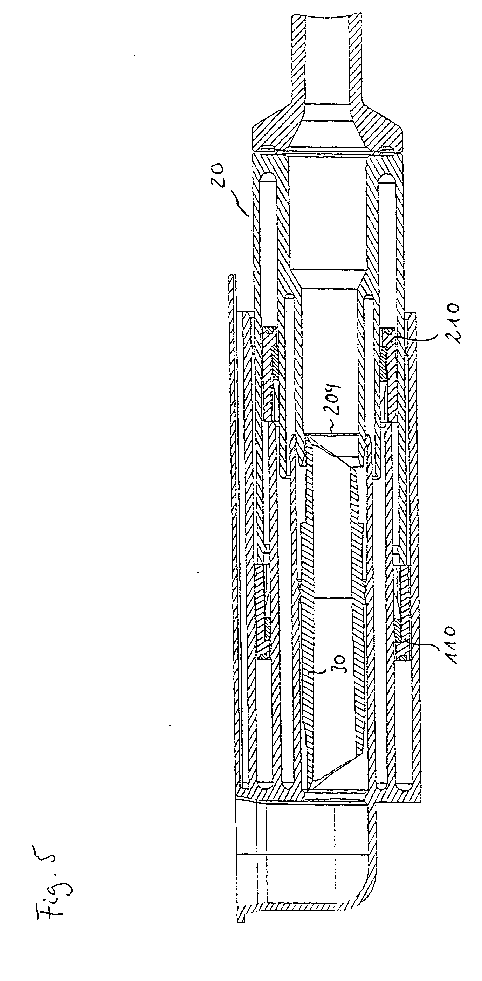

Werden die Konnektorelemente aus der in Fig. 4 dargestellten Position weiter zusammengeführt, ergibt sich der in Fig. 5 dargestellte Zustand. Hier sind zum einen die Verschlußteile 110, 210 weiter in die entsprechenden Ringspalte eingeführt und zum anderen liegt nunmehr der Durchstoßkörper 30 an dem Absperrelement 204 des Konnektorelementes 20 an. Das Absperrelement 204 ist als Spritzgußmembran ausgeführt, die vorzugsweise aus Polypropylen besteht und gegen alle bisher bekannten Sterilisationsverfahren beständig ist. Eine derartige Membran stellt nicht nur einen Auslaufschutz bzw. einen Schutz vor Bakterien dar, sondern bietet dauerhaft Dichtigkeit bis hin zu mehreren Bar Differenzdruck. Das Absperrelement 204 weist eine Stärke von ca. 0,2 mm auf.

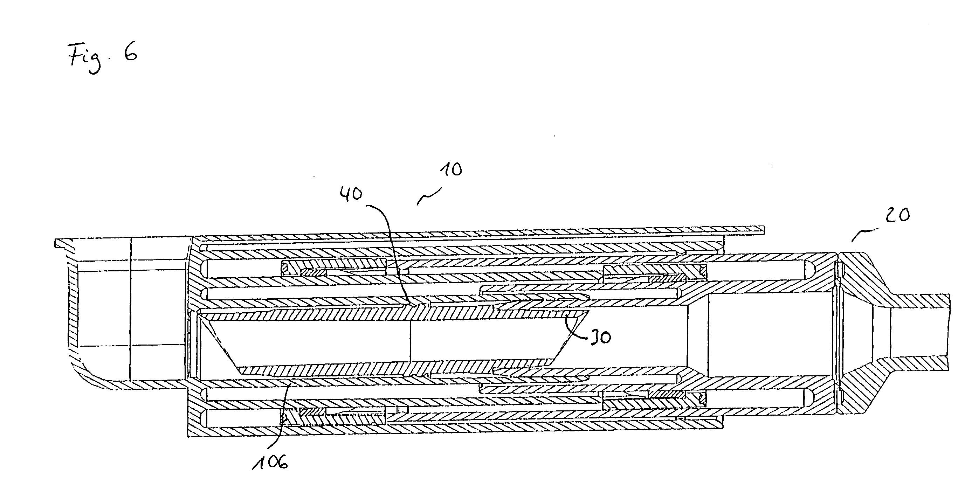

Fig. 6 zeigt die Position beider Konnektorelemente 10, 20 nach dem Durchstoßen des Absperrelementes durch den Durchstoßkörper 30. Der Durchstoßkörper 30 ist ein beidseitig angespitztes Spritzgießbauteil, welches bei der Herstellung in das Rohr 106 eingeschoben wird und dort durch eine Arretierung 40 gegen Herausfallen gesichert ist. Der Durchstoßkörper 30 läßt sich sehr kostengünstig in Vielfach-Spritzgießwerkzeugen herstellen, wobei für die konstruktive Gestaltung zahlreiche Freiheiten gegeben sind. In der in Fig. 6 dargestellten Position befindet sich der Durchstoßkörper 30 noch immer in der in der Mitte des Durchstoßkörpers 30 dargestellten Arretierung 40 in dem Rohr 106 des Konnektorelementes 10.

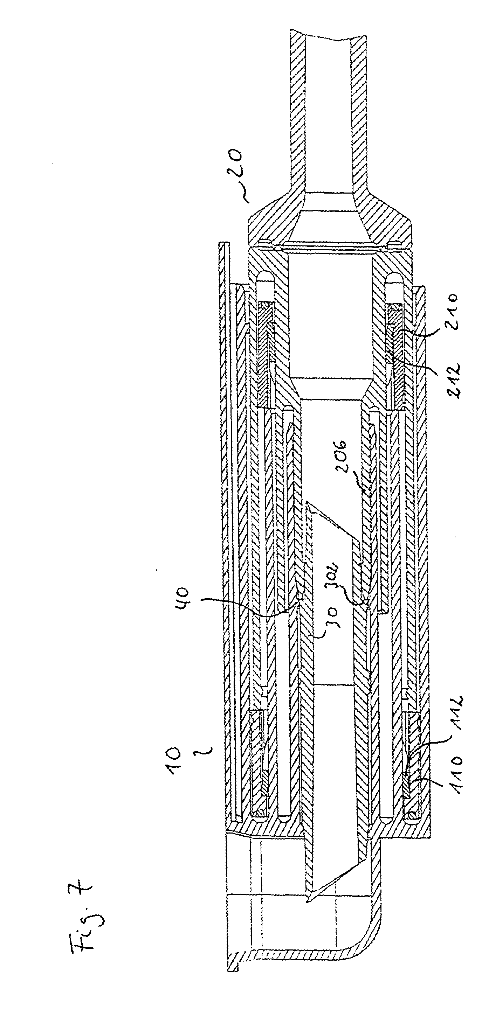

Fig. 7 zeigt den Endzustand, in dem der Durchstoßkörper 30 nun aus seiner Arretierung 40 herausbewegt wurde und dabei die als Absperrelement 104 dienende Membran durchtrennt hat. Die Bewegung des Durchstoßkörpers 30 wird dadurch bewirkt, daß der umlaufende Vorsprung 302 durch den Endbereich des Rohres 206 berührt wird, wodurch der Durchstoßkörper in die in Fig. 7 dargestellte Position bewegt wird. Der Durchstoßkörper 30 übernimmt somit die Aufgabe, die beiden Absperrelemente zu durchstoßen und anschließend so aufzuweiten, daß ein ausreichender Strömungsquerschnitt freigegeben wird.

Aus Fig. 7 wird weiterhin ersichtlich, daß die Verschlußteile 110, 210 bzw. die geöffneten Membranen 112, 212 in den Endbereichen der jeweiligen Ringspalte angeordnet sind. Die Membranen 112, 212 bestehen aus einer ca. 0,6 mm starken Silikonmembran, die mit dem Polyamid-Spritzgußrand in einem speziellen Zwei-Komponenten-Spritzgießwerkzeug zusammen hergestellt werden. Typische Abmessungen des einzuführenden Konnektorelementes 20 sind ein Außendurchmesser von 12,6 mm sowie eine Einstecktiefe von 34 mm. Um zu erreichen, daß sich die Membranen 112, 212 nach der Dekonnektion der Konnektorelemente wieder schließen, können Rückstellelemente vorgesehen sein, die eine entsprechende auf die Verschlußteile 110, 210 wirkende Kraft ausüben. Derartige Rückstellelemente können beispielsweise als Federn ausgeführt sein, die in den Ringspalten zwischen dem Gehäuse 108, 208 und den Rohrstutzen 116, 216 angeordnet sind. Ebenso ist es denkbar, Federn vorzusehen, die durch seitliche Schlitze der dem Ringspalt benachbarten Wandung geführt werden und die nach der Dekonnektion eine Rückführung der Verschlußelemente 110, 210 bewirken.

Das erfindungsgemäße Konnektionssystem bietet den Vorteil, daß dieses originalitätsverschlossen und dicht auf beiden Seiten ausgeführt ist.

Für den sicheren Konnektionsvorgang spielt es keine Rolle, ob die Konnektoren flüssigkeits- oder gasgefüllt sind. Nachträgliche Konnektionen während beispielsweise eines Disposable-Einsatzes sind möglich. Dabei spielt die Flußrichtung keine Rolle.

Ferner ist das Konnektionssystem modular aufgebaut. Ohne Maßänderungen sind die Konnektorelemente mit oder ohne Verschlußteil, mit oder ohne Absperrelement oder mit teilweiser Verschluß- und Absperrelementbestückung herstellbar. Bei Disposables mit hoher Komplexität, bei denen nach dem aktuellen Stand der Polypropylen-Spritzgußtechnik das Spritzen mehrerer Membranen in einem Arbeitsgang noch nicht gelingt, sind Konstruktionsvarianten einsetzbar, bei denen Polypropylenoder Elastomermembranen nachträglich an die vorgesehenen Stellen eingebaut werden können.

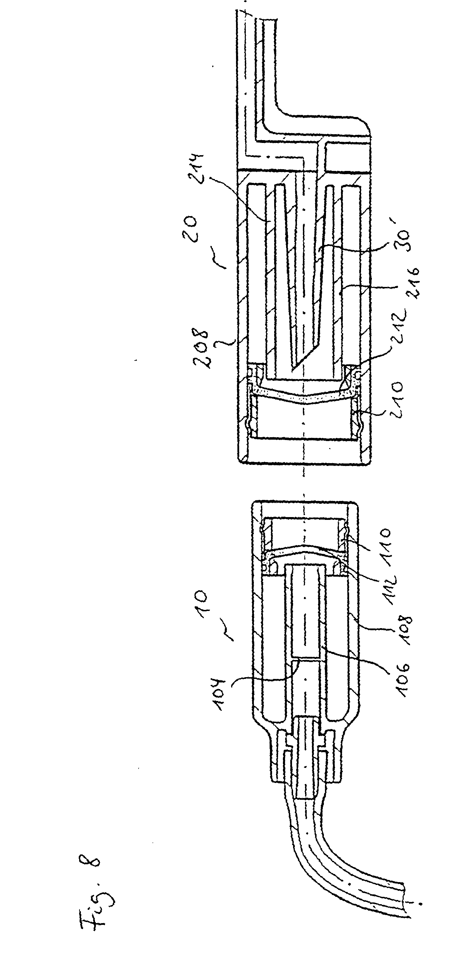

Fig. 8 zeigt zwei Konnektorelemente 10, 20 mit den Verschlußteilen 110, 210 sowie mit daran angeordneten Membranen 112, 212.

Das Konnektorelement 10 weist in dem Rohrstutzen 106 ein Absperrelement 104 auf, das aus einer Spritzgußmembran besteht. Das Absperrelement 104 wird bei der Konnektion der Konnektorelemente 10, 20 durch den im Konnektorelement 20 angeordneten Dorn 30' geöffnet und auf diese Weise eine fluiddichte Verbindung hergestellt.

Vor der Öffnung des Absperrelementes 104 erfolgt eine Verschiebung der Verschlußteile 110, 210 in die entsprechenden Ringspalte, wobei sich der Ringspalt des Konnektorelementes 10 zwischen dem Rohrstutzen 106 und dem Gehäuse 108 und des Konnektorelementes 20 zwischen dem als Rohrstutzen 216 ausgeführten Öffnungselement 214 und dem Gehäuse 208 erstreckt. Beim Zusammenführen der Konnektoren 10, 20 erfolgt zunächst ein Verschieben des Verschlußteils 210 durch das Gehäuse 108 in den entsprechenden Ringspalt, wobei die Membran 212 durch den Endbereich des Rohrstutzens 216 erfindungsgemäß geöffnet wird und dadurch der Dorn 30' mit der Membran 212 nicht in Berührung kommt. Daraus ergibt sich der Vorteil, daß dieser unabhängig von einer auf der Innenseite der Membran 212 befindlichen Kontamination in einem sterilen Zustand gehalten werden kann. Im Anschluß an die teilweise Verschiebung des Verschlußteils 210 erfolgt die Öffnung des Verschlußteils 110 bzw. der Membran 112 durch einen stirnseitigen Kontakt des Verschlußteils 110 mit dem Endbereich des Rohrstutzens 216. Dabei wird das Verschlußteil 110 in den Ringspalt eingeführt, die Membran 112 geöffnet und der Dorn 30' in den Rohrstutzen 106 eingeführt bis ein Öffnen des Absperrelementes 104 erfolgt.

Bei der in Fig. 8 dargestellten Ausführungsform sind die Verschlußteile 110, 210 bzw. die Membranen 112, 212 als Silikon-Polyamid-Verbund-Spritzgußteil ausgeführt, wobei die etwa 1 mm starken Membranen nachträglich schlitz- oder kreuzförmig durchstochen werden. Diese Anordnung führt dazu, daß Bakterien abgehalten werden, und verhindert bis etwa 0,05 Bar das Auslaufen von Flüssigkeit. Andererseits öffnet sich die Membran relativ leicht beim Verschieben.

Das Konnektorelement 10 kann als mit einem Beutel verbundenes Gebe-Konnektorelement und das Konnektorelement 20 als mit einem Disposable verbundenen Empfangs-Konnektorelement ausgeführt sein. Erfindungsgemäß ergibt sich ein Fingerberührschutz durch die zurückversetzte Anordnung der Membranen 112, 212 beutel- und disposableseitig. Darüber hinaus liegt beutelseitig bis zur Konnektion in Form des Absperrelementes 104 eine hermetisch dichte Durchstechmembran sowie ein ultraschallgeschweißter Schlauchstutzenadapter vor.

Die in Fig. 8 dargestellten Konnektorelemente 10, 20 weisen ein elastomerfreies Dorn-Konnektionssystem auf. Dabei bieten die Verschlußteile 110, 210 mit den Membranen 112, 212 einen wirksamen Schutz gegen beispielsweise Anhusten oder feste oder flüssige Partikel sowie einen Auslaufschutz und umfassen eine Originalitäts-Verschlußfunktion in beiden Konnektorelementen 10, 20.

## Patentansprüche

1. Konnektorelement (10, 20) insbesondere zum Verbinden von Schläuchen, Kanülen und Kathetern mit einem Bereich (102, 202) zur Führung eines durchströmenden Mediums, mit einem relativ zu diesem Bereich (102, 202) bewegbaren Verschlussteil(110, 210), durch das der Bereich (110, 202) gegenüber der das Konnektorelement (10, 20) umgebenden Atmosphäre abdichtbar ist, und das von diesem Bereich (102, 202) aufgrund eines zwischen dem Bereich (102, 202) und dem Verschlussteil (110, 210) angeordneten Öffnungselementes (114, 214) beabstandet ist,
**dadurch gekennzeichnet,**
**dass** das Verschlussteil (110, 210) einen Grundkörper umfasst, über dessen Querschnitt sich die Membran (112, 212) erstreckt und der einen zur Konnektionsöffnung des Konnektorelements (10, 20) gerichteten Endbereich aufweist, wobei die Membran (112, 212) von diesem Endbereich des Grundkörpers entfernt angeordnet ist.

2. Konnektorelement (10, 20) nach Anspruch 1, **dadurch gekennzeichnet, daß** der Grundkörper zylindrisch ausgeführt ist und die Membran (112, 212) in einem der Endbereiche des Grundkörpers angeordnet ist.

3. Konnektorelement (10, 20) nach Anspruch 1 oder 2, **dadurch gekennzeichnet, daß** die Membran (112, 212) zum Zwecke der Öffnung bei Konnektion einen geradlinigen oder kreuzförmigen Schlitz aufweist.

4. Konnektorelement (10, 20) nach einem oder mehreren der Ansprüche 1 bis 3, **dadurch gekennzeichnet, daß** die Membran (112, 212) als Silikonmembran ausgeführt ist.

5. Konnektorelement (10, 20) nach einem oder mehreren der Ansprüche 1 bis 4, **dadurch gekennzeichnet, daß** der Bereich (102, 202) zur Führung eines durchströmenden Mediums einen Rohrstutzen (106, 206) umfaßt.

6. Konnektorelement (10, 20) nach einem oder mehreren der Ansprüche 1 bis 5, **dadurch gekennzeichnet, daß** das Öffnungselement (114, 214) einen Rohrstutzen (116, 216) darstellt, der den Bereich (102, 202) zur Führung des Mediums umgibt.

7. Konnektorelement (10, 20) nach Anspruch 5 und 6, **dadurch gekennzeichnet, daß** der Bereich (102, 202) zur Führung des Mediums sowie das Öffnungselement (114, 214) durch konzentrisch angeordnete Rohrstutzen (106, 116, 206, 216) gebildet werden, wobei der das Öffnungselement (114, 214) bildende Rohrstutzen (116, 216) mit dem den Bereich (106, 206) zur Führung des Mediums begrenzenden Rohrstutzen (106, 206) abschließt oder geringfügig über diesen übersteht.

8. Konnektorelement (10, 20) nach einem oder mehreren der Ansprüche 1 bis 7, **dadurch gekennzeichnet, daß** das Konnektorelement (10, 20) ein Gehäuse (108, 208) aufweist und der Bereich (102, 202) zur Führung des Mediums sowie das Öffnungselement (114, 214) in dem Gehäuse (108, 208) aufgenommen sind.

9. Konnektorelement (10, 20) nach Anspruch 8, **dadurch gekennzeichnet, daß** sich zwischen Gehäuse (108, 208) und Öffnungselement (114, 214) ein Ringspalt erstreckt, in dem das Verschlußteil (110, 210) bewegbar aufgenommen ist.

10. Konnektorelement (10, 20) nach Anspruch 9, **dadurch gekennzeichnet, daß** das Verschlußteil (110, 210) in dem Gehäuse (108, 208) mittels einer Arretierung (50) fixierbar ist.

11. Konnektorelement (10, 20) nach einem oder mehreren der Ansprüche 1 bis 10, **dadurch gekennzeichnet, daß** der Bereich (102, 202) zur Führung des Mediums durch ein Absperrelement (104, 204) verschlossen ist.

12. Konnektorelement (10, 20) nach Anspruch 11, **dadurch gekennzeichnet, daß** ein relativ zu dem Absperrelement (104, 204) bewegbarer Durchstoßkörper (30) vorgesehen ist, der derart angeordnet ist, daß das Absperrelement (104, 204) durch den Durchstoßkörper (30) geöffnet werden kann.

13. Konnektorelement (10, 20) nach Anspruch 12, **dadurch gekennzeichnet, daß** der Bereich (102, 202) zur Führung des Mediums durch einen Rohrstutzen (106, 206) gebildet wird und der Durchstoßkörper (30) in dem Rohrstutzen (106) angeordnet ist.

14. Konnektorelement (10, 20) nach Anspruch 13, **dadurch gekennzeichnet, daß** der Durchstoßkörper (30) einen Vorsprung (302) aufweist, der mit einem Rohrstutzen (206) verbindbar ist.

15. Konnektorelement (10, 20) nach einem oder mehreren der Ansprüche 11 bis 14, **dadurch gekennzeichnet, daß** das Absperrelement (104, 204) als Spritzgußmembran ausgeführt ist.

## Claims

1. A connector element (10, 20), in particular for connecting hoses, cannulae and catheters, comprising a region (102, 202) for guiding a medium flowing therethrough, and a closure portion (110, 210) which is movable relative to said region (102, 202) and by which the region (102, 202) can be sealed off with respect to the atmosphere surrounding the connector element (10, 20) and which is spaced from said region (102, 202) by virtue of an opening element (114, 214) arranged between the region (102, 202) and the closure portion (110, 210), **characterised in that** the closure portion (110, 210) includes a main body, over the cross-section of which the diaphragm (112, 212) extends and which has an end region directed towards the connection opening of the connector element (10, 20), wherein the diaphragm (112, 212) is arranged remote from said end region of the main body.

2. A connector element (10, 20) according to claim 1 **characterised in that** the main body is cylindrical and the diaphragm (112, 212) is arranged in one of the end regions of the main body.

3. A connector element (10, 20) according to claim 1 or claim 2 **characterised in that** the diaphragm (112, 212) has a straight or cross-shaped slot for the purposes of opening upon connection.

4. A connector element (10, 20) according to one or more of claims 1 to 3 **characterised in that** the diaphragm (112, 212) is in the form of a silicone diaphragm.

5. A connector element (10, 20) according to one or more of claims 1 to 4 **characterised in that** the region (102, 202) for guiding a medium flowing therethrough has a tubular portion (106, 206).

6. A connector element (10, 20) according to one or more of claims 1 to 5 **characterised in that** the opening element (114, 214) represents a tubular portion (116, 216) which surrounds the region (102, 202) for guiding the medium.

7. A connector element (10, 20) according to claim 5 and claim 6 **characterised in that** the region (102, 202) for guiding the medium and the opening element (114, 214) are formed by concentrically arranged tubular portions (106, 116, 206, 216), wherein the tubular portion (116, 216) forming the opening element (114, 214) terminates with or projects slightly beyond the tubular portion (106, 206) delimiting the region (106, 206) for guiding the medium.

8. A connector element (10, 20) according to one or more of claims 1 to 7 **characterised in that** the connector element (10, 20) has a housing (108, 208) and the region (102, 202) for guiding the medium and the opening element (114, 214) are accommodated in the housing (108, 208).

9. A connector element (10, 20) according to claim 8 **characterised in that** extending between the housing (108, 208) and the opening element (114, 214) is an annular gap in which the closure portion (110, 210) is movably accommodated.

10. A connector element (10, 20) according to claim 9 **characterised in that** the closure portion (110, 210) can be fixed in the housing (108, 208) by an arresting means (50).

11. A connector element (10, 20) according to one or more of claims 1 to 10 **characterised in that** the region (102, 202) for guiding the medium is closed by a shut-off element (104, 204).

12. A connector element (10, 20) according to claim 11 **characterised in that** there is provided a piercing body (30) which is movable relative to the shut-off element (104, 204) and which is so arranged that the shut-off element (104, 204) can be opened by the piercing body (30).

13. A connector element (10, 20) according to claim 12 **characterised in that** the region (102, 202) for guiding the medium is formed by a tubular portion (106, 206) and the piercing body (30) is arranged in the tubular portion (106).

14. A connector element (10, 20) according to claim 13 **characterised in that** the piercing body (30) has a projection (302) which can be connected to a tubular portion (206).

15. A connector element (10, 20) according to one or more of claims 11 to 14 **characterised in that** the shut-off element (104, 204) is in the form of an injection moulded diaphragm.

## Revendications

1. Elément de raccord (10, 20), en particulier pour le raccordement de tuyaux, canules et cathéters avec une zone (102, 202) de conduite d'un liquide traversant, avec une pièce d'obturation mobile (110, 210) par rapport à cette zone (102, 202), pièce par le biais de laquelle la zone (102, 202) peut être étanchéifiée par rapport à l'atmosphère entourant l'élément de raccord (10, 20), et qui est située à distance de cette zone (102, 202) en raison d'un élément d'ouverture (114, 214) disposé entre la zone (102, 202) et la pièce d'obturation (110, 210).
**caractérisé en ce que**
la pièce d'obturation (110, 210) comprend une partie principale à travers la section de laquelle s'étend une membrane (112, 212) et qui comporte une zone terminale tournée vers l'ouverture de raccordement de l'élément de raccord (10, 20), la membrane (112, 212) étant disposée loin de cette zone terminale de la partie principale.

2. Elément de raccord (10, 20) selon la revendication 1, **caractérisé en ce que** la partie principale est réalisée en cylindre et que la membrane (112, 212) est disposée dans l'une des zones terminales de la partie principale.

3. Elément de raccord (10, 20) selon la revendication 1 ou 2, **caractérisé en ce que** la membrane (112, 212) comprend, à des fins d'ouverture lors du raccordement, une fente rectiligne ou en croix.

4. Elément de raccord (10, 20) selon les revendications 1 à 3, **caractérisé en ce que** la membrane (112, 212) est réalisée en tant que membrane en silicone.

5. Elément de raccord (10, 20) selon les revendications 1 à 4, **caractérisé en ce que** la zone (102, 202) de conduite d'un fluide traversant comprend un embout de tube (106, 206).

6. Elément de raccord (10, 20) selon les revendications 1 à 5, **caractérisé en ce que** l'élément d'ouverture (114, 214) représente un embout de tube (116, 216) qui entoure la zone (102, 202) de conduite du fluide.

7. Elément de raccord (10, 20) selon les revendications 5 et 6, **caractérisé en ce que** la zone (102, 202) de conduite du fluide ainsi que l'élément d'ouverture (114, 214) sont formés par des embouts de tube (106, 116, 206, 216) disposés de manière concentrique, l'embout de tube (116, 216) formant l'élément d'ouverture (114, 214) se terminant par l'embout de tube (106, 206), délimitant la zone (106, 206) de conduite du fluide, ou dépassant légèrement celui-ci.

8. Elément de raccord (10, 20) selon une ou plusieurs des revendications 1 à 7, **caractérisé en ce que** l'élément de raccord (10, 20) comprend un boîtier (108, 208) et que la zone (102, 202) de conduite du fluide ainsi que l'élément d'ouverture (114, 214) sont logés dans le boîtier (108, 208).

9. Elément de raccord (10, 20) selon la revendication 8, **caractérisé en ce que**, entre le boîtier (108, 208) et l'élément d'ouverture (114, 214) s'étend une fente annulaire dans laquelle est logée la pièce d'obturation (110, 210) de manière mobile.

10. Elément de raccord (10, 20) selon la revendication 9, **caractérisé en ce que** la pièce d'obturation (110, 210) peut être fixée dans le boîtier (108, 208) au moyen d'un dispositif d'arrêt (50).

11. Elément de raccord (10, 20) selon une ou plusieurs des revendications 1 à 10, **caractérisé en ce que** la zone (102, 202) de conduite du fluide est obturée par un élément de fermeture (104, 204).

12. Elément de raccord (10, 20) selon la revendication 9, **caractérisé en ce qu'**un élément de perçage (30) mobile par rapport à l'élément de fermeture (104, 204) est prévu, lequel élément de perçage est disposé de telle sorte que l'élément de fermeture (104, 204) puisse être ouvert par l'élément de perçage (30).

13. Elément de raccord (10, 20) selon la revendication 12, **caractérisé en ce que** la zone (102, 202) de conduite du fluide est formée par un embout de tube (106, 206) et que l'élément de perçage (30) est disposé dans l'embout de tube (106).

14. Elément de raccord (10, 20) selon la revendication 13, **caractérisé en ce que** l'élément de perçage (30) comporte une saillie (302) qui est raccordable à un embout de tube (206).

15. Elément de raccord (10, 20) selon une ou plusieurs des revendications 11 à 14, **caractérisé en ce que** l'élément de fermeture (104, 204) est réalisé en tant que membrane moulée par injection.
